# FASCICULE DE BREVET EUROPEEN

(11) **EP 1 278 468 B1**
(45) Date de publication et mention de la délivrance du brevet: **26.10.2005**
(21) Numéro de dépôt: 00946047.8
(22) Date de dépôt: 30.06.2000
(51) Int. Cl.: A61B 17/70

(54) **CONNECTEUR TRANSVERSAL POUR SYSTEME D'OSTEOSYNTHESE RACHIDIENNE**
QUERVERBINDER FÜR WIRBELSÄULENOSTEOSYNTHESE
TRANSVERSE CONNECTOR FOR SPINAL OSTEOSYNTHESIS SYSTEM

(30) Priorité: 01.07.1999 FR 9908496
(43) Date de publication de la demande: 29.01.2003
(73) Titulaire: Spinevision S.A., 75012 Paris (FR)
(72) Inventeur: VANACKER, Gérard, F-94100 Saint-Maur (FR)
(74) Mandataire: Breese, Pierre
(86) Numéro de dépôt international: PCT/FR2000/001870
(87) Numéro de publication internationale: WO 2001/001872

(56) Documents cités:
- EP-A- 0 446 092
- EP-A- 0 676 177
- EP-A- 0 689 799
- US-A- 5 584 831
- US-A- 5 984 923

## Description

La présente invention concerne le domaine de l'ostéosynthèse du rachis, et plus particulièrement le domaine de la correction de l'alignement des vertèbres par un système comprenant des tiges de correction, des crochets aptes à être fixés sur les vertèbres et des barres de liaison transverses.

De tels systèmes forment un cadre de correction rigide en torsion.

A titre d'exemple de l'art antérieur, on se reportera au brevet européen EP95910695, publié sous le numéro EP750477. Ce brevet décrit notamment un crochet de fixation pour connecter entre elles une tige d'un système d'ostéosynthèse rachidienne avec une barre transversale rigide et pour serrer la tige contre la barre, ledit crochet de fixation comprenant :
- un corps ;
- un passage défini par des portions marginales dans ledit corps pour la réception de la barre, ledit passage possédant une première hauteur au moins égale à l'épaisseur de la barre, s'étendant sur toute la longueur dudit corps, s'ouvrant librement vers l'extérieur à une extrémité et se terminant à une extrémité dans une ouverture définie dans ledit corps, et possédant une seconde hauteur dépassant l'épaisseur de la barre ;
- une portion de bande courbe s'étendant depuis ledit corps pour que vienne s'y loger une tige, ladite ouverture étant prévue dans une zone de connexion entre ledit corps et ladite portion de bande courbe ;
- un trou taraudé défini dans ledit corps, qui s'ouvre dans ledit passage et qui est positionné de telle sorte que l'axe dudit trou taraudé se trouve à proximité d'une portion terminale libre de ladite portion de bande courbe ;
- une vis de serrage insérée par filet de vis dans ledit trou taraude pour serrer une barre dans ledit passage sur une tige logée dans ladite portion de bande courbe ;
- par lequel la vis de serrage, lorsqu'elle serre ladite barre, exerce une force de serrage le long d'une ligne qui est décalée à proximité de la portion terminale libre de ladite portion de bande courbe par rapport à l'axe central de la tige de façon à provoquer un mouvement pivotant de la barre autour de ladite tige.

Un autre brevet européen connu publié sous le numéro EP446092 décrit un autre dispositif de liaison transversale rigide entre deux tiges d'ostéosynthèse rachidienne. Ce dispositif comprend deux éléments de fixation constitués chacun par un crochet adapté pour pouvoir coiffer une barre transversale rigide de manière coulissante, équipé de moyens de blocage sur la barre. Ce crochet est formé d'un corps et de deux lames distantes d'un intervalle de largeur correspondant à celle de la barre, et une portée d'appui du crochet sur la barre est ménagée sur le corps entre les lames, lesquelles s'étendent de chaque côté de la barre lorsque le crochet chevauche cette dernière. Deux crochets combinés à une barre rectangulaire, forment un dispositif de liaison transverse relativement simple et rapide à mettre en place et présentant une forte rigidité en torsion et en flexion.

Les systèmes de liaisons transverses de l'art antérieur nécessitent un parallélisme parfait des deux éléments de liaison constitués par des tiges ou par des plaques. Dans le cas où les tiges ne sont pas parallèles, le chirurgien doit cintrer l'élément de liaison transverse pour adapter les éléments de fixation sur les éléments de liaison.

Les solutions de l'art antérieur autorisent une fixation libre dans le plan frontal ou dans le plan sagittal, ou dans une combinaison de ces deux plans de rotation. Elles ne permettent toutefois pas la totalité des orientations relatives désirées. Elles ne permettent pas d'éviter l'opération d'adaptation par torsion des éléments transverses ou des dispositions défavorables à la rigidité globale du système d'ostéosynthèse par rapport à la proéminence des implants.

Le but de la présente invention est de remédier à ces inconvénients en proposant un connecteur pour un système d'ostéosynthèse permettant de réaliser un système de correction de grande rigidité après serrage, mais autorisant une correction de l'alignement des éléments transverses dans les différents plans, et garantissant un blocage simultané de l'ensemble des tiges et éléments de correction. A cet effet, l'invention concerne dans son acception la plus générale un connecteur et un élement de liaison transverse présentant une tige transverse pour système d'ostéosynthèse ledit connecteur étant destiné à assurer une liaison entre deux tiges d'un système d'ostéosynthèse rachidienne, constitué par un crochet adapté pour pouvoir coiffer de manière coulissante l'extrémité d'une barre transversale rigide, ce crochet étant équipé de moyens de blocage sur ladite barre, caractérisé en ce que le corps du crochet présente un logement semi-cylindrique orienté sensiblement selon un premier axe, pour recevoir une tige de section circulaire, un deuxième logement présentant un axe sensiblement perpendiculaire au premier axe, ce logement débouchant dans le logement semi-cylindrique et étant destiné à recevoir l'une des extrémités sensiblement sphériques de la tige transverse, et un troisième alésage débouchant dans le deuxième logement et présentant un filetage pour recevoir une vis de serrage venant exercer une pression sur l'extrémité sphérique de la tige transverse, ladite extrémité sphérique venant en appui sur la tige cylindrique.

Un avantage important d'un tel connecteur est de permettre la mise en tension ou en compression du cadre par glissement des connecteurs le long des tiges, après la mise en place du cadre. Un autre avantage est qu'un tel connecteur évite la présence de parties protubérantes sous la tige (antérieure à la tige par rapport au patient), ce qui facilite la mise en place sur l'os.

Avantageusement, le connecteur pour système d'ostéosynthèse selon l'invention est destiné à assurer une liaison rigide entre deux tiges d'un système d'ostéosynthèse rachidienne.

Avantageusement également, le deuxième logement débouche par un tronçon conique évasé pour autoriser un cône de mobilité de la tige transverse avant serrage de la vis de blocage.

De préférence, l'ouverture du logement semi-cylindrique s'étend sur environ 180°.

Selon un mode de réalisation particulier, le fond du logement cylindrique présente une forme générale de fer à cheval.

L'invention concerne dans un mode de réalisation préféré également un système d'ostéosynthèse comprenant au moins une tige transverse, au moins une tige de correction et au moins un crochet équipé de moyens de blocage sur ladite tige et ladite barre transverse, caractérisé en ce que la barre transverse présente des extrémités hémisphériques et en ce que le corps du crochet présente un logement semi-cylindrique orienté sensiblement selon un premier axe, pour recevoir une tige de section circulaire, un deuxième logement présentant un axe sensiblement perpendiculaire au premier axe, ce logement débouchant dans le logement semi-cylindrique et étant destiné à recevoir l'extrémité sensiblement sphérique d'une tige transverse, et un troisième alésage débouchant dans le deuxième logement et présentant un filetage pour recevoir une vis de serrage venant exercer une pression sur l'extrémité sphérique de la tige transverse, ladite extrémité sphérique venant en appui sur la tige cylindrique.

L'invention sera mieux comprise à la lecture de la description qui suit, se référant aux dessins annexés où :

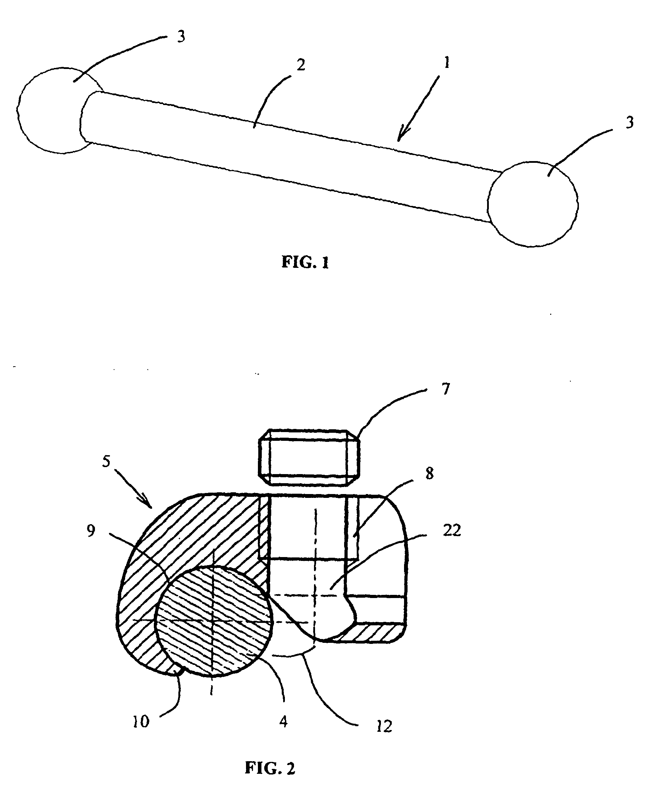

- la figure 1 représente une vue de la tige de liaison transverse ;
- la figure 2 représente une vue en coupe de la pièce de liaison ;
- la figure 3 représente une vue selon un plan de coupe perpendiculaire au précédent, de ladite pièce de liaison ;
- les figures 4 et 5 représentent deux vues d'un système selon l'invention.

L'élément de liaison transverse (1) présente globalement une forme d'haltère. Il est destiné à relier les éléments de liaison longitudinaux constitués par des tiges ou des plaques. Il présente un segment cylindrique médian formant une tige transverse (2) prolongé à chaque extrémité (3) par une partie hémisphérique.

La tige transverse (2) peut être rectiligne ou au contraire arqué pour permettre une adaptation plus aisée à l'anatomie du patient, par exemple une forme oméga. Il présente une section circulaire ou quelconque.

Une telle forme en oméga permet de passer au-dessus des apophyses épineuses de la vertèbre instrumentée. L'entre axe des deux extrémités (3) sphériques - et donc la longueur de la tige transverse (2) rectiligne ou arquée - diffère suivant l'écartement des éléments de liaison constitués par des tiges ou des plaques. Dans un système complet, on proposera avantageusement une variété d'éléments de liaison présentant des écartements différents.

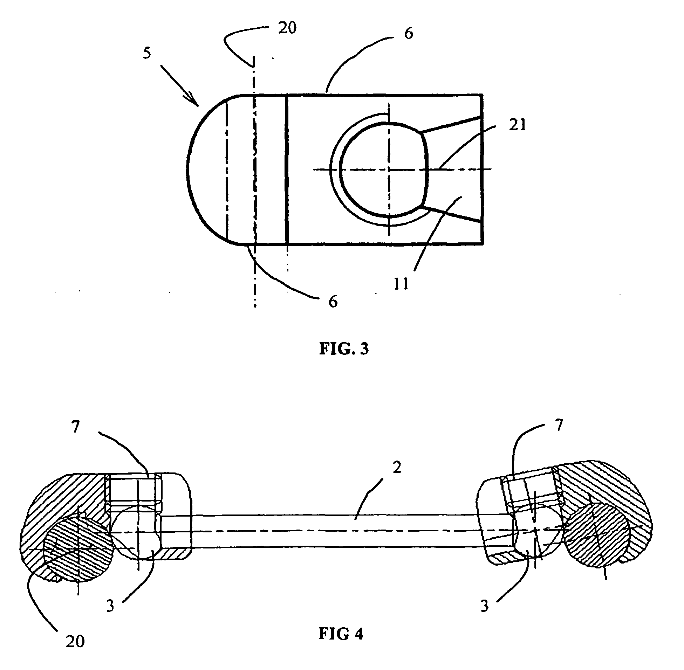

Les extrémités (3) sphériques de l'élément de liaison transverse viennent en contact avec des éléments de liaisons longitudinaux assurant la correction du rachis et son réalignement, comme représenté en figures 3 et 4.

Le connecteur (5) est formé par une noix représentée en figure 2. Ils présentent un logement semi-cylindrique (10) orienté sensiblement selon un premier axe (20), pour recevoir une tige (4) de section circulaire. Il présente un trou taraudé (8) destiné à recevoir une vis de pression (7) de manière à solidariser l'ensemble de façon rigide. Ce même connecteur peut recevoir également sur ses facettes, par exemple la face latérale (6), des trous de préhensions afin de faciliter la manipulation du connecteur, voire même de l'ensemble du système de liaison transverse. Le connecteur (5) présente par ailleurs une gorge (9) recevant l'élément de liaison longitudinal. La forme de la gorge (9) est légèrement différente de celle de la tige (4) afin d'assurer une auto-stabilité de l'assemblage.

L'élément de liaison formé par la tige (4) est préférentiellement une tige postérieure ou de liaison circulaire, ce qui autorise une rotation de celle-ci autour de son axe. Les implants sont formés par des crochets, des vis pédiculaires ou des plaques de fixations au sacrum.

Le connecteur (5) se charge sur la tige selon une direction postérieure au patient, qui représente dans ce cas l'élément de liaison transverse. Le connecteur (5) présente un trou taraudé (8) désaxé par rapport à l'axe (20) de la tige (4). Ce trou taraudé (8) a une première fonction qui est de recevoir la vis de serrage (7) assurant le blocage du système.

Il a également une deuxième fonction qui est de laisser le passage libre pour l'introduction de l'extrémité (3) sphérique de la pièce transverse. A cet effet, la section de l'alésage du trou taraudé (8) est au moins égale à la section de l'extrémité (3) sphérique, et débouche dans l'ouverture du deuxième logement (11) conique destinée au passage de la tige transverse (2) de la pièce transverse.

Lorsque l'ensemble des composants du système sont positionnés, les vis de serrage sont introduites dans les alésages des connecteurs.

Le serrage provoque l'appui de l'extrémité de la vis sur l'extrémité (3) sphérique de la pièce transverse, cette extrémité (3) sphérique venant en appui sur la tige (4). Elle assure ainsi le blocage de la tige (4) dans son logement semi-cylindrique. Le serrage de la vis (7) assure ainsi simultanément le blocage de toutes les pièces passant par le connecteur.

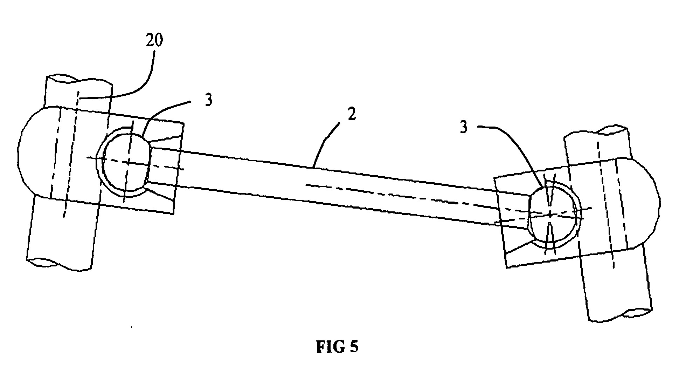

La forme conique du deuxième logement (11) autorise un débattement de la pièce transverse, comme représenté en figure 4 et 5.

## Revendications

1. Connecteur et un élément de liaison transverse (1) présentant une tige (2) transverse pour système d'osthéosynthèse, ledit connecteur étant destiné à assurer une liaison entre l'élément transverse (1) et une tige (4) de correction rigide, ledit connecteur étant constitué par un crochet adapté pour pouvoir coiffer de manière coulissante la tige (4), ce crochet étant équipé de moyens de blocage sur ladite tige, **caractérisé en ce que** ladite tige (2) transverse comprenant des extrémités sensiblement sphériques, et **en ce que** le corps du crochet présente
un logement semi-cylindrique (9) orienté sensiblement selon un premier axe (20), pour recevoir ladite tige (4) de correction de section circulaire,
un deuxième logement (11) présentant un axe (21) sensiblement perpendiculaire au premier axe (20), ce logement (11) débouchant dans le logement (9) semi-cylindrique et étant destiné à recevoir l'une des extrémités (3) sensiblement sphériques de la tige transverse (2) de l'élément de liaison transverse (1), et
un troisième logement (22) débouchant dans le deuxième logement (11) et présentant un filetage pour recevoir une vis de serrage (7) adaptée à exercer une pression sur l'extrémité (3) sphérique de la tige transverse (2), ladite extrémité (3) sphérique pour venir en appui sur la tige (4) cylindrique.

2. Connecteur et élément de liaison transverse pour système d'osthéosynthèse selon la revendication 1, **caractérisés en ce que** le deuxième logement (11) débouche par un tronçon conique évasé pour autoriser un cône de mobilité de la tige transverse (2) avant serrage de la vis de blocage (7).

3. Connecteur et un élément de liaison transverse (1) présentant une tige transverse pour système d'osthéosynthèse selon la revendication 1 ou 2 **caractérisés en ce que** le logement semi-cylindrique (9) s'étend sur environ 180°.

4. Connecteur et un élément de liaison transverse (1) présentant une tige transverse pour système d'osthéosynthèse selon l'une quelconque des revendications 1 à 3, **caractérisés en ce que** le fond du logement semi-cylindrique (9) présente une forme générale de fer à cheval.

5. Connecteur et un élément de liaison transverse (1) présentant une tige transverse pour système d'osthéosynthèse selon l'une quelconque des revendications 1 à 4, **caractérisés en ce qu'**une face latérale (6) comporte des trous de préhensions afin de faciliter la manipulation du connecteur.

6. Connecteur et un élément de liaison transverse (1) présentant une tige transverse pour système d'osthéosynthèse selon l'une quelconque des revendications 1 à 5, **caractérisés en ce que** le logement (22) présente un alésage pour recevoir la vis de serrage (7) qui présente une section au moins égale à la section de l'extrémité sphérique (3) de l'élément de liaison transverse (1) afin de permettre le passage de ladite extrémité par cet alésage.

7. Système d'ostéosynthèse comprenant au moins un élément de liaison transverse (1) présentant une tige transverse (2) selon l'une quelconque des revendications 1 à 6, **caractérisé en ce qu'**il comprend au moins une tige (4) de correction.

## Patentansprüche

1. Stecker und ein transversales Verbindungselement (1) vorzeigend einen transversalen Stab (2) für System der Ostheosynthese, besagter Stecker bestimmt dazu eine Verbindung zu sichern zwischen dem transversalen Element (1) und einem steifen Verbesserungsstab (4), besagter Stecker besteht aus einem Haken angepasst um auf gleitende Weise den Stab (4) zu umfassen, dieser Haken ist ausgestattet mit Blockiermitteln auf besagtem Stab, **dadurch gekennzeichnet, dass** besagter transversaler Stab (2) beinhaltet merklich sphärische Endteile und dass der Körper des Hakens präsentiert
eine halbzylindrische Lagerung (9) merklich orientiert entsprechend einer ersten Achse (20), um besagten Verbesserungsstab (4) von kreisförmigem Querschnitt zu erhalten,
eine zweite Lagerung (11) welche eine merklich senkrechte Achse (21) zur ersten Achse (20) präsentiert, diese Lagerung (11) mündet in der halbzylindrischen Lagerung (9) und ist bestimmt dazu zu erhalten eine der merklich sphärischen Endteile (3) des transversalen Stabes (2) des transversalen Verbindungselements (1), und
eine dritte Lagerung (22) mündent in die zweite Lagerung (11) und präsentierend ein Gewinde um eine Festziehschraube (7) zu erhalten angepasst um einen Druck auszuüben auf die sphärische Endteile (3) des transversalen Stabes (2), besagte sphärische Endteile (3) auf Anschlag zu kommen auf den zylindrischen Stab (4).

2. Stecker und transversales Verbindungselement für System der Ostheosynthese nach Anspruch 1, **dadurch gekennzeichnet, dass** die zweite Lagerung (11) durch einen konischen ausladenden Abschnitt um zu erlauben einen Mobilitätskegel des transversalen Stabes (2) vor dem Festziehen der Blockierschraube (7) mündet.

3. Stecker und ein transversales Verbindungselement (1) präsentierend einen transversalen Stab für System der Ostheosynthese nach den Ansprüchen 1 oder 2 **dadurch gekennzeichnet, dass** die halb-zylindrische Lagerung (9) sich auf ungefähr 180° ausdehnt.

4. Stecker und ein transversales Verbindungselement (1) präsentierend einen transversalen Stab für System der Ostheosynthese nach einem der Ansprüche 1 bis 3 **dadurch gekennzeichnet, dass** der Boden der halb-zylindrischen Lagerung (9) eine allgemeine Form eines Hufeisens präsentiert.

5. Stecker und ein transversales Verbindungselement (1) präsentierend einen transversalen Stab für System der Ostheosynthese nach einem der Ansprüche 1 bis 4 **dadurch gekennzeichnet, dass** eine seitliche Seite (6) beinhaltet Greiflöcher um die Manipulation des Steckers zu erleichtern.

6. Stecker und ein transversales Verbindungselement (1) präsentierend einen transversalen Stab für System der Ostheosynthese nach einem der Ansprüche 1 bis 5 **dadurch gekennzeichnet, dass** die Lagerung (22) eine Bohrung enthält um die Festziehschraube (7) zu erhalten welche präsentiert einen Querschnitt wenigstens gleich dem Querschnitt der sphärischen Endteile (3) des transversalen Verbindungselements (1) um das Durchführen besagten Endteils durch diese Bohrung zu erlauben.

7. System der Ostheosynthese beinhaltend wenigstens ein transversales Verbindungselement (1) präsentierend einen transversalen Stab (2) nach einem der Ansprüche 1 bis 6 **dadurch gekennzeichnet, dass** es beinhaltet wenigstens einen Verbesserungsstab (4).

## Claims

1. Connector and a transverse connecting element (1) with a transverse rod (2) for an ostheosynthesis system, the said connector designed to make a link between the transverse element (1) and a rigid correction rod (4), the said connector being composed of a hook adapted to fit over the rod (4) and free to slide on it, this hook being provided with means of locking onto the said rod, **characterised in that** the said transverse rod (2) comprises approximately spherical ends and **in that** the body of the hook comprises:
- a semi-cylindrical housing (9) facing approximately along a first axis (20), to hold the said circular-shaped correction rod (4),
- a second housing (11) with an axis (21) approximately perpendicular to the first axis (20), this housing (11) opening up in the semi-cylindrical housing (9) and being designed to receive one of the approximately spherical ends (3) of the transverse rod (2) of the transverse connecting element (1), and
- a third housing (22) opening up into the second housing (11) and with a thread adapted to hold a tightening screw (7) and apply a pressure on the spherical end (3) of the transverse rod (2), so that the said spherical end (3) comes into contact with the cylindrical rod (4).

2. Connector and transverse connecting element for an ostheosynthesis system according to claim 1, **characterised in that** the second housing (11) opens up into a flared conical segment to allow the transverse rod (2) to move within a mobility cone before the locking screw (7) is tightened.

3. Connector and a transverse connecting element (1) with a transverse rod for an ostheosynthesis system according to claim 1 or 2, **characterised in that** the semi-cylindrical housing (9) extends over about 180°.

4. Connector and a transverse connecting element (1) with a transverse rod for an ostheosynthesis system according to any one of claims 1 to 3, **characterised in that** the bottom of the semi-cylindrical housing (9) is in the general shape of a horseshoe.

5. Connector and a transverse connecting element (1) with a transverse rod for an ostheosynthesis system according to any one of claims 1 to 4, **characterised in that** a side face (6) is provided with gripping holes to facilitate handling of the connector.

6. Connector and a transverse connecting element (1) with a transverse rod for an ostheosynthesis system according to any one of claims 1 to 5, **characterised in that** the housing (22) is provided with a reaming in which the tightening screw (7) fits and that has a section equal to at least the section of the spherical end (3) of the transverse connecting element (1) so as to allow the said end to pass through this reaming.

7. Ostheosynthesis system comprising at least one transverse connecting element (1) with a transverse rod (2) according to any one of claims 1 to 6, **characterised in that** it comprises at least one correction rod (4).
